Europäisches Patentamt

(19) European Patent Office

Office européen des brevets

(11) Veröffentlichungsnummer: **0 040 666**
**B1**

(12) # EUROPÄISCHE PATENTSCHRIFT

(45) Veröffentlichungstag der Patentschrift:
28.09.83

(21) Anmeldenummer: **81101008.1**

(22) Anmeldetag: **13.02.81**

(51) Int. Cl.³: **C 07 C 57/04,** C 07 C 69/54,
C 07 C 51/25, C 07 C 51/377,
C 07 C 67/317 // C07C47/22,
C07C45/65, B01J12/00,
B01J15/00, B01J19/18,
B01J35/04, C07B3/00

(54) Katalytisches Oxydations- und Dehydrierungsverfahren und Verwendung einer dafür geeigneten Umsetzungsvorrichtung.

(30) Priorität: **23.05.80 DE 3019731**
**22.12.80 DE 3048455**

(43) Veröffentlichungstag der Anmeldung:
**02.12.81 Patentblatt 81/48**

(45) Bekanntmachung des Hinweises auf die Patenterteilung:
**28.09.83 Patentblatt 83/39**

(84) Benannte Vertragsstaaten:
**BE DE FR GB IT NL**

(56) Entgegenhaltungen:
**CH-A-566 807**
**DE-A-2 324 164**
**DE-A-2 645 363**
**DE-A-2 834 358**
**DE-A-2 942 359**
**DE-C-844 737**
**FR-A-1 310 748**

(73) Patentinhaber: **Röhm GmbH, Kirschenallee**
**Postfach 4242, D-6100 Darmstadt 1 (DE)**

(72) Erfinder: **Siegert, Hermann-Josef, Dr., Inselstrasse 21,**
**D-6100 Darmstadt (DE)**
Erfinder: **Bub, Günther Karl-Heinz, Dr., Auf dem**
**Backenberg 13, D-4630 Bochum-Querenburg (DE)**
Erfinder: **Regner, Hans, Dr., Griesheimer Strasse 7,**
**D-4370 Marl (DE)**

Katalytisches Oxydations- und Dehydrierungsverfahren und Verwendung
einer dafür geeigneten Umsetzungsvorrichtung

Die Erfindung betrifft ein kontinuierliches Verfahren zur Herstellung von Oxydations- oder Dehydrierungsprodukten von organischen Verbindungen durch Umsetzung eines die organische Verbindung im Gaszustand und Sauerstoff und gegebenenfalls Moderatorgas enthaltenden Frischgases durch heterogene Katalyse an einem Katalysator, der in einem Reaktionsraum angeordnet ist, zu einem Reaktionsgas, das Oxydations- bzw. Dehydrierungsprodukte der organischen Verbindung sowie gegebenenfalls unverbrauchten Sauerstoff enthält. Als Moderatorgas werden solche unter den Verfahrensbedingungen gasförmigen Stoffe verstanden, die an der Umsetzung mit dem Sauerstoff nicht teilnehmen, und zwar weil sie entweder unter den Reaktionsbedingungen gegenüber Sauerstoff inert sind, wie z. B. Wasserdampf oder Stickstoff, oder weil sie in einem stöchiometrischen Überschuß gegenüber dem Sauerstoff vorliegen. In diesem Fall kann es sich bei dem Moderatorgas um einen Teil der gasförmigen organischen Verbindung handeln. Der gebräuchlichste Reaktortyp für derartige Verfahren sind Rohrbündelreaktoren, die den Katalysator in einem Bündel von parallel angeordneten Rohren, die von einem wärmeaufnehmenden Medium umspült werden, enthalten. Das Frischgas wird in einem vorgeschalteten Wärmetauscher auf eine zur Durchführung der Reaktion ausreichende Temperatur vorgewärmt und durchströmt dann die Reaktorrohre. Dabei erwärmt es sich in der Regel weiter, jedoch wird ein Großteil der Reaktionswärme an das Kühlmedium abgegeben.

Um eine hohe Selektivität zu erreichen, wird eine weitgehend isotherme Fahrweise angestrebt. Dieses Ziel ist in der Praxis nicht oder nur unter hohem Aufwand erreichbar, weil es ein sehr großes Verhältnis von Kühlfläche zu Reaktorvolumen voraussetzt. Der Katalysator müßte in einer sehr großen Zahl langer und dünner Rohre angeordnet werden. Das erhöht die Apparatekosten, erschwert das Einfüllen und Entleeren des Katalysators und verursacht einen hohen Strömungswiderstand.

Um bei begrenzter Kühlfläche einen für die Selektivität schädlichen Temperaturanstieg vom Eingang zum Ausgang des Reaktors und einen hohen Temperaturgradienten vom Zentrum zur Peripherie des einzelnen Reaktorrohres zu vermeiden, mischt man dem Gasgemisch aus der umzusetzenden organischen Verbindung und Sauerstoff erhebliche Mengen eines Moderatorgases bei. Es erhöht einerseits die Wärmekapazität je Volumeneinheit des umzusetzenden Gases und vermindert andererseits die je Raumeinheit des Katalysatorbettes entwickelte Wärmemenge.

Die Mitverwendung von großen Mengen an Moderatorgas bringt schwerwiegende Nachteile mit sich. Man benötigt erhebliche Mengen der Moderatorgase selbst, sehr große Reaktorräume, große Leitungsquerschnitte, leistungsfähige Gebläse zur Bewegung des Reaktionsgases, große Wärmetauscher und erhebliche Energie- und Kühlmittelmengen zum Aufheizen des Moderatorgases vor der Umsetzung und zum Abkühlen nach der Umsetzung. Nach der Umsetzung sind Stofftrennverfahren erforderlich, die wegen der großen Gasvolumina aufwendig sind.

Als Beispiel für Verfahren dieser Art sei die katalytische Dehydrierung von Isobuttersäure, Isobutyraldehyd oder Methacrolein zu Methacrylsäure nach den Verfahren der DE-OS 2 633 593 und 2 722 375 genannt. Als Moderatorgase werden Wasserdampf und Stickstoff, der letztere in wesentlich größerer Menge als der mit dem Luftsauerstoff eingebrachte Luftstickstoff, eingesetzt. Das Volumenverhältnis von Moderatorgas zu der umzusetzenden organischen Verbindung liegt etwa zwischen 17 und 45. Man erreicht Selektivitäten von 57 bis 84%.

Aus der DE-OS 2 324 164 ist ein Reaktor für katalytische Reaktionen in der Gasphase bekannt, bei dem das umzusetzende Frischgas durch einen Strahlmischer in den Reaktorraum eingeleitet wird, wobei ein Teil des aus dem Reaktionsraum ausgetretenen Reaktionsgases mitgerissen und in den Reaktionsraum zurückgeleitet wird. Die katalytische Reaktion wird adiabatisch durchgeführt. Als Vorteile werden ein verminderter Druckabfall im Reaktor und ein geringerer Energiebedarf infolge der teilweisen Ausnutzung der Reaktionswärme zum Vorwärmen des Frischgases genannt. Nachteilig ist die Notwendigkeit, das Frischgas zu komprimieren, um es mit hoher Strömungsgeschwindigkeit durch eine Düse in den Strahlmischer zu leiten, mit rückgeführtem Reaktionsgas zu vermischen und durch die Katalysatorschicht zu drücken, sowie der große Reaktorraum im Vergleich zum Katalysatorraum, was Nebenreaktionen in der Gasphase begünstigt. Der Reaktor soll u. a. für die Oxydation von Äthylen zu Äthylenoxid oder von Propylen zu Acrolein oder Acrylsäure geeignet sein.

Eine Vorrichtung zur Durchführung katalytischer Gasreaktionen ist in der DE-PS 844 737 beschrieben. Das Reaktionsgas wird mit einem Gebläse im Kreislauf über den Katalysator und über Wärmetauscher geführt, während Frischgas zugemischt und ein Teil des Reaktionsgases zur Aufarbeitung abgezogen wird. Die Vorrichtung ist zur Synthese von Kohlenwasserstoffen vorgeschlagen worden. Die Durchführung von Oxydations- bzw. Dehydrierungsverfahren in dieser Vorrichtung ist nicht bekannt.

Für reaktionskinetische Untersuchungen im Laboratoriumsmaßstab werden sogenannte Differentialreaktoren verwendet. Darin wird das zu untersuchende Gasgemisch mit einer hohen Umwälzgeschwindigkeit im Kreislauf über einen Katalysator geleitet, wobei bei jedem Umlauf immer nur ein äußerst geringer Umsatz stattfindet. Dadurch ist es möglich, Betriebszustände an verschiedenen Punkten eines Rohrreaktors im Laboratorium zu simulieren und den Einfluß der

2

**0 040 666**

Betriebsparameter zu untersuchen. Deshalb muß bei diesen Untersuchungen grundsätzlich das gleiche Gasgemisch wie bei dem zugehörigen großtechnischen Verfahren eingesetzt werden. Für die Untersuchung von katalytischen Oxydations- bzw. Dehydrierungsverfahren bedeutet dies die Mitverwendung gleichgroßer Mengen an Moderatorgas wie im Rohrreaktor.

Die Durchführung von technischen Herstellungsverfahren mittels Differentialreaktoren entsprechender Größe ist nicht bekannt.

Der Erfindung liegt die Aufgabe zugrunde, bei einem kontinuierlichen Verfahren zur Herstellung von Oxydations- oder Dehydrierungsprodukten von organischen Verbindungen durch Umsetzung eines die organische Verbindung im Gaszustand und Sauerstoff und gegebenenfalls Moderatorgas enthaltenden Frischgases durch heterogene Katalyse zu einem Reaktionsgas, das Oxydations- bzw. Dehydrierungsprodukte der organischen Verbindung und gegebenenfalls unverbrauchte Anteile der organischen Verbindung und des Sauerstoffs enthält, den Anteil an Moderatorgas im Frischgas ohne Nachteil für die Selektivität zu vermindern. Die Aufgabe wird erfindungsgemäß bei einem Verfahren, bei dem man einen Teil des aus dem Reaktionsraum austretenden Reaktionsgases mit dem Frischgas, das eine unter der Umsetzungstemperatur liegende Eintrittstemperatur hat, vermischt und das entstandene Gasgemisch in den Reaktionsraum, wo der Katalysator angeordnet ist, einleitet, in der Weise gelöst, daß man das Gasgemisch bzw. das Reaktionsgas mittels einer mechanisch angetriebenen Umwälzvorrichtung durch den Reaktionsraum leitet und ein Frischgas mit nicht mehr als 12 Volumenteilen Moderatorgas je Volumenteil der gasförmigen organischen Verbindung einsetzt.

In der Regel beschränkt sich die Zufuhr von Moderatorgas auf den zusammen mit dem Luftsauerstoff eingeführten Luftstickstoff. Allgemein liegt der bevorzugte Moderatorgasanteil bei 2 bis 6 Volumenteilen je Volumenteil der organischen Verbindung.

Je höher die Umwälzgeschwindigkeit ist, um so kleiner ist der Anteil des Frischgases an dem durch den Reaktionsraum strömenden Gasgemisch und um so näher kommt man dem Betriebszustand des ideal durchmischten Reaktors. Dies ist ein theoretischer Grenzzustand, bei dem die Konzentrationsunterschiede der Ausgangs- und Endstoffe bei einem Durchgang durch den Reaktionsraum beliebig klein werden. In diesem Zustand herrschen an jeder Stelle des Reaktionsraumes die gleichen Betriebsbedingungen, insbesondere die gleiche Temperatur. Es ist zur Aufrechterhaltung einer hohen Selektivität keineswegs erforderlich, sich dem Zustand des ideal durchmischten Reaktors weitgehend anzunähern. Bereits bei einem Anteil des rückgeführten Reaktionsgases von 80 Volumenprozent, bezogen auf das Gesamtvolumen des in den Reaktionsraum eintretenden Gasgemisches, wird eine hohe Selektivität erreicht. Vorzugsweise wird dieser Anteil zwischen 80 und 98% gehalten, d. h. das Frischgas wird mit der etwa 4- bis 50fachen Menge an Rücklaufgas vermischt. Das genaue Volumenverhältnis von Rücklaufgas zu Frischgas ist oft schwierig zu messen. Bei der allmählichen Verminderung des Rücklaufgasanteils, z. B. durch Drosselung der Leistungsaufnahme der Umwälzvorrichtung, tritt meistens an einem bestimmten Punkt ein starker Abfall der Selektivität ein. Es genügt, dicht über diesem Punkt zu arbeiten, wobei das Rücklaufverhältnis nicht genau bekannt zu sein braucht. Selbstverständlich wird im Reaktionsraum dauernd so viel Frischgas umgesetzt, wie in den Reaktor eintritt.

Infolge der Vermischung mit dem rückgeführten Reaktionsgas (Rücklaufgas) vermindert sich das Konzentrationsgefälle für die umzusetzende organische Verbindung und für den Sauerstoff vom Eingang bis zum Ausgang des Reaktionsraumes im gleichen Verhältnis wie das Volumenverhältnis von Rücklaufgas zum Frischgas. Wird z. B. bei dem erfindungsgemäßen Verfahren ein Gesamtumsatz von 70% erzielt und beträgt das Volumenverhältnis des Frischgases zum Rücklaufgas 1 : 10, so tritt innerhalb des Reaktionsraumes nur noch ein Konzentrationsgefälle von 7% für die organische Verbindung auf. Bei einem Volumenverhältnis von 1 : 50 würde sich das Konzentrationsgefälle auf 1,4% erniedrigen.

Die drastische Verminderung des Konzentrationsgefälles ist gerade bei Oxydationen und oxydativen Dehydrierungen von großer Bedeutung. Im Prinzip können die organischen Verbindungen mit Sauerstoff stufenweise bis zur vollständigen Verbrennung zu Kohlendioxid reagieren. Durch die Wahl geeigneter Katalysatoren kann die Bildung bestimmter gewünschter Zwischenstufen gefördert werden, wenn auch eine weitergehende Verbrennung zu höheren Oxydations- bzw. Dehydrierungsprodukten nie völlig unterdrückt werden kann. Die Selektivität, also der Anteil der erwünschten Umsetzungsprodukte an der Gesamtheit der überhaupt entstehenden Umsetzungsprodukte, erreicht unter ganz bestimmten Temperatur- und Konzentrationsbedingungen ein Maximum. Ein ideal durchmischter Reaktor könnte genau auf diesen Betriebspunkt eingestellt werden. In der Praxis kommt man diesem Zustand nur in dem Maße nahe, wie durch die Einstellung des Frischgas-Rücklaufgas-Verhältnisses die Temperatur- und Konzentrationsunterschiede im Reaktionsraum eigeengt werden können.

Jede Abweichung vom optimalen Betriebspunkt fördert die Bildung unerwünschter Nebenprodukte. Vor allem ein Temperaturanstieg über den günstigsten Wert erweist sich als schädlich, weil dabei in zunehmendem Maß unkontrollierte Verbrennung auftritt. Die hohe Verbrennungswärme, die dabei entsteht, erhöht die Temperatur weiter und läßt das unerwünschte Reaktionsgeschehen lawinenartig anschwellen. Es bilden sich sogenannte »hot spots«, in denen neben dem Verlust der Ausbeute meist auch ein irreversibler Verlust der Katalysatoraktivität eintritt.

3

Im klassischen Rohrreaktor wird diese Gefahr der Überhitzung durch große Mengen an Moderatorgasen vermindert, wenn auch um den Preis der eingangs erwähnten Nachteile. Die unempfindlichen Moderatorgase, wie Stickstoff, Kohlendioxid oder Wasserdampf, erhöhen auch bei einer lokalen Überhitzung nicht die Wärmeerzeugung. Beim Verfahren der Erfindung treten nun an die Stelle der Moderatorgase die im Kreislauf geführten Ausgangs- und Endstoffe, die keineswegs inert sind. Die Überhitzungsgefahr ist bei diesem Gasgemisch also besonders groß. Die Moleküle dieser Stoffe müssen den Reaktionsraum beispielsweise zehnmal, in manchen Fällen hundertmal oder noch öfter durchlaufen, wobei sie nur einmal reagieren sollen. Es war zu befürchten, daß dabei mehr unerwünschte Nebenprodukte entstehen, als durch die Beschränkung auf einen engen Konzentrations- und Temperaturbereich vermieden werden.

Überraschenderweise ist das nicht der Fall. Selbst wenn der Anteil des Moderatorgases auf die Menge an Stickstoff beschränkt wird, die zusammen mit Luftsauerstoff unvermeidlich eingeführt wird, erhält man erfindungsgemäß gleichhohe Selektivitäten wie mit extrem großen Moderatorgasmengen bei der Umsetzung im Rohrreaktor. Selbst beim Arbeiten mit reinem Sauerstoff, d. h. beim vollständigen Verzicht auf Moderatorgas, wird die Selektivität kaum beeinträchtigt. Als besonders vorteilhaft erweist sich der Verzicht auf Wasserdampf als Moderatorgas.

Während bei der oxidativen Dehydrierung von Isobuttersäure zu Methacrylsäure die Beimischung von mindestens 2 Volumenteilen Wasserdampf je Volumenteil des Ausgangsstoffes als unerläßlich gilt, wird erfindungsgemäß mit Vorteil ohne jeden Wasserdampfzusatz gearbeitet. Andererseits ist es nicht erforderlich, die Verbrennungsluft oder das Frischgas vor dem Einleiten in den Reaktor zu trocknen. Bei der Dehydrierung entsteht ohnehin stets etwas Wasserdampf, der bei der Aufarbeitung des Reaktionsgases abgetrennt werden muß. Während die Abtrennung von größeren Mengen Wasser aus dem Reaktionsprodukt, beispielsweise wegen der Existenz von Azeotropen, mit einem beträchtlichen Verfahrensaufwand verbunden ist, läßt sich die begrenzte Menge an Reaktionswasser, sofern es bei der Weiterverarbeitung des Reaktionsproduktes überhaupt stört, einfacher entfernen, wenn das Frischgas weniger als 1 Mol, vorzugsweise weniger als 0,5 Mol und besonders bevorzugt weniger als 0,1 Volumenteile Wasserdampf je Volumenteil der gasförmigen organischen Verbindung enthält.

Das Verfahren der Erfindung dient zur Herstellung von Oxydations- und Dehydrierungsprodukten von organischen Verbindungen. Als Oxydationsprodukte werden im engeren Sinne Verbindungen mit einem höheren Sauerstoffgehalt als die Ausgangsverbindung bezeichnet, wobei der Sauerstoff in Hydroxy-, Epoxy-, Carbonyl-, Carboxyl- oder Anhydridgruppen enthalten sein kann. Im weiteren Sinne gehören auch die Produkte der Ammonoxydation, also Acryl- und Methacrylnitril, dazu. Dehydrierungsprodukte sind solche, die weniger Wasserstoffatome als die Ausgangsverbindung bzw. eine vergrößerte Zahl von olefinischen oder Carbonyldoppelbindungen enthalten. Vorzugsweise enthält die als Ausgangsstoff eingesetzte organische Verbindung mindestens 2 und höchstens 5 Kohlenstoffatome, wobei Verbindungen mit einer aliphatischen $C_3$-Gruppe oder einer Iso-$C_4$-Gruppe besonders bevorzugt sind; dazu gehören Propylen, Isopropylalkohol und Acrolein als $C_3$-Körper und Isobutylen, Methacrolein, Isobuttersäure und ihre niederen Alkylester, besonders Methylisobutyrat, sowie Isobutyraldehyd als Iso-$C_4$-Körper. Typische Umsetzungen, für die sich das Verfahren der Erfindung anwenden läßt, sind die Oxidation bzw. oxydative Dehydrierung von

Propylen zu Acrolein oder zu Acrylsäure
Propylen zu Propylenoxid
Propylen mit Ammoniak zu Acrylnitril
Isobutylen mit Ammoniak zu Methacrylnitril
Isobutylen zu Methacrolein oder zu Methacrylsäure
Isobutyraldehyd zu Isobuttersäure oder zu Methacrylsäure
Isobuttersäure zu Methacrylsäure
Isobuttersäure-alkylestern zu Methacrylsäure-alkylestern

sowie ferner die Oxydation von

Blausäure zu Dicyan
Äthylen zu Äthylenoxid
Äthylen zu Acetaldehyd
Buten-1 oder -2 zu Maleinsäureanhydrid
Benzol zu Maleinsäureanhydrid

Eine bevorzugte Gruppe von Reaktionen sind die, welche von Isobutyraldehyd oder insbesondere von Isobuttersäure oder Isobuttersäuremethylester ausgehen und zu Methacrylsäure bzw. Methylmethacrylat führen.

Sauerstoff wird i. allg. wenigstens in der stöchiometrischen Menge, berechnet auf die organische Verbindung, und bevorzugt in einem Überschuß bis zum Doppelten der stöchiometrischen Menge eingesetzt. Die Größe der stöchiometrischen Menge hängt von der überwiegend ablaufenden Reaktion ab. Bei der Umsetzung von Isobuttersäure zu Methacrylsäure beträgt sie 1/2 Mol, bei der Umsetzung von Isobutyraldehyd zum gleichen Endprodukt 1 Mol Sauerstoff je Mol der Ausgangsverbindung.

Der Sauerstoff wird vorzugsweise in Form von Luft eingesetzt und bringt in diesem Falle das 4fache Volumen an Stickstoff als Moderatorgas ein. Das Verfahren kann auch mit an Sauerstoff angereicherter Luft oder mit reinem Sauerstoff durchgeführt werden. Für Ammonoxydationsverfahren wird zusätzlich Ammoniak im stöchiometrischen Verhältnis mitverwendet, wobei für die genaue Dosierung auf die an Rohrreaktoren ermittelten Ergebnisse zurückgegriffen werden kann.

Der Reaktionsablauf wird — wie vom Rohrreaktor bekannt — entscheidend von der Wahl des Katalysators beeinflußt, das gilt besonders für die Höhe des Umsatzes und der Selektivität. Grundsätzlich können im Verfahren der Erfindung die gleichen Katalysatoren angewendet werden, die sich im Rohrreaktor für die gleiche Umsetzung bewährt haben. Als Beispiele seien Heteropolysäuren, wie Phosphormolybdänsäure oder Phosphorwolframsäure und deren Verbindungen mit anderen Metallen, z. B. Vanadin, Eisen, Alkalimetallen, besonders Cäsium, genannt, die für die oxydative Dehydrierung von Isobutyraldehyd oder Isobuttersäure zu Methacrylsäure oder den entsprechenden Estern geeignet sind. Die Herstellung gut geeigneter Katalysatoren für diese Reaktion ist z. B. in der DE-OS 2 633 593 und der DE-OS 2 722 375 beschrieben. Weitere bewährte Katalysatoren für die erfindungsgemäß durchzuführenden Reaktionen enthalten die Elemente Ag, Cu, Cr, Mo, W, Mn, V, Fe, Co, Ni, Pd, Pt in metallischer Form oder in Form der Oxide oder anderer Verbindungen als Hauptbestandteil und gegebenenfalls weitere Elemente, wie Alkali- und Erdalkalimetalle, Antimon, Wismut oder Tellur, als Nebenbestandteile.

In der Regel wird der Katalysator auf einem Träger, wie Silikagel, Kieselgur oder Aluminiumoxid eingesetzt. Der Katalysator ist in einem Reaktionsraum — vorzugsweise fest — angeordnet. Er kann z. B. in Form eines grobkörnigen Schüttgutes vorliegen. Bei feinerer Körnung kommt auch ein Wirbelbettkatalysator in Betracht.

Mit Vorteil wendet man Katalysatoranordnungen mit besonders niedrigem Druckverlust für das durchströmende Gas an. Der Druckverlust ist um so geringer, je gröber die Katalysatormasse gestückelt, je dünner die durchströmte Schicht ist und je geradliniger die vom Gas durchströmten Kanäle sind. Besonders vorteilhaft ist ein sogenannter Wabenkatalysator. Dort ist die aktive Katalysatorsubstanz in einer Vielzahl von weitgehend geradlinigen Kanälen angeordnet, die mit sehr geringem Druckverlust durchströmt werden. Der Wabenträgerkörper wird nach dem Extrusions- oder dem Wickelverfahren, z. B. aus Siliciumoxid, Siliciumcarbid, Aluminiumoxid, Titanoxid, Mullit, Spondumen oder Cordierit hergestellt und mit der aktiven Katalysatorsubstanz belegt. Diese ist auf der Oberfläche der Kanalwandungen und bei porösem Wandmaterial auch in deren Innern angeordnet.

Die Umsetzungstemperatur kann in der Regel auf einen engen Bereich beschränkt werden. Vorzugsweise wird das Verfahren adiabatisch durchgeführt, d. h. die gesamte Reaktionswärme wird mit dem Reaktionsprodukt aus dem Reaktor ausgetragen. Die Reaktionstemperatur ergibt sich dann aus der Temperatur und Wärmekapazität des Frischgases, der Reaktionswärme und der Wärmekapazität des austretenden Reaktionsgases. Die geringe Erwärmung des Reaktionsgases im Reaktionsraum wird durch die Zumischung des kühleren Frischgases zu dem Rücklaufgas ausgeglichen. Eine streng adiabatische Durchführung ist in der Regel nicht erforderlich, d. h. Wärmeverluste durch Strahlung oder durch Wärmeabgabe an die umgebende Luft brauchen nicht durch aufwendige Mittel verhindert zu werden, zumal wenn die Reaktion genügend Wärme freisetzt, um die optimale Temperatur zu erreichen. Die Feinsteuerung der Temperatur im Reaktor geschieht am besten durch die Regulierung der Frischgastemperatur. Daneben besteht die Möglichkeit der nicht-adiabatischen Betriebsweise, bei der der rückgeführte Teilstrom des Reaktionsgases über einen Wärmetauscher geleitet wird.

Die Eintrittstemperatur des Frischgases kann bei normaler Raumtemperatur oder darüber oder — z. B. nach Entspannung eines Druckgases — darunter liegen. Sofern das Frischgas kondensierbare Dämpfe enthält, wie z. B. Isobutyraldehyd oder Isobuttersäure, muß die Temperatur des Frischgases so hoch gewählt werden, daß keine Kondensation eintritt. Das Frischgas kann in mehreren getrennten Gasströmen von gegebenenfalls unterschiedlicher Temperatur eingeleitet werden; beispielsweise können die organische Verbindung bei ihrem Siedepunkt und Luft bei der herrschenden Außentemperatur über getrennte Leitungen zugeführt werden.

In vielen Fällen sind Umsetzungstemperaturen im Bereich von 200 bis 400°C gut geeignet, wobei der Temperaturunterschied innerhalb des Reaktors in weit engeren Grenzen liegt. In der Regel überschreitet er nicht einen Betrag von 50° und mit besonderem Vorteil von 10°. Bei hoher Umwälzrate kann der Temperaturanstieg vom Eingang bis zum Ausgang des Reaktionsraumes in der Größenordnung von einem oder wenigen Graden liegen.

Bei einigen der durchführbaren Oxydations- bzw. Dehydrierungsreaktionen, insbesondere bei einem hohen Umsatz, kann die abzuführende überschüssige Reaktionswärme bei nicht-adiabatischer Verfahrensweise so hoch sein, daß der apparative Aufwand für Wärmetauscher erheblich ins Gewicht fällt. Schon bei geringen Anteilen einer Totalverbrennung bis zum Kohlendioxid steigt die tatsächliche Reaktionswärme erheblich über den theoretischen Wert für die Hauptreaktion an.

Um in diesem Falle einen unerwünscht hohen Temperaturanstieg zu vermeiden oder die erforderliche Kühlleistung herabzusetzen, kann die organische Verbindung zum Teil oder vollständig in flüssiger Form in das Frischgas bzw. den mit dem Frischgas zu vermischenden Teilstrom des Reaktionsgases eingespeist und verdampft werden. Dieser Gasstrom wird durch den Entzug der Verdampfungswärme und durch die Wärmekapazität der organischen Verbindung beträchtlich abgekühlt. Gleichzeitig wird die entzogene Wärme ohne zusätzlichen Bedarf an aufwendigen technischen Vorrichtungen vollständig nutzbar gemacht.

Bei adiabatischer Fahrweise oder bei vorgegebener Kühlleistung kann die Eintrittstemperatur im Reaktor dadurch auf den günstigsten Wert eingestellt werden, daß man das Verhältnis der im Gaszustand und der in flüssiger Form zugeführten Anteile der organischen Verbindung variiert. Die Reaktionswärme kann jedoch auch so groß sein, daß bei vollständiger Zugabe der organischen Verbindung in flüssiger Form noch eine Kühlung des Reaktionsgases mit einem Wärmetauscher notwendig ist, um einen unerwünschten Temperaturanstieg des Reaktionsgases zu vermeiden.

Wenn beispielsweise Isobuttersäure zu Methacrylsäure dehydriert wird, so entsteht pro Mol eingesetzter Isobuttersäure bei 92%igem Umsatz und 74% Selektivität und einem Kreislaufverhältnis von $v=10$ eine gesamte Reaktionsenthalpie von 47,1 kcal. Unter dem Kreislaufverhältnis wird der Quotient aus dem Volumenstrom im Kreislauf und dem Volumenstrom des abströmenden Gases verstanden. Wenn die Isobuttersäure dampfförmig bei ihrem Siedepunkt eingesetzt wird und Luft als Oxydationsmittel verwendet wird, so werden zur Aufheizung des Frischgases auf die Reaktionstemperatur 21,6 kcal/Mol verbraucht. Um im kontinuierlichen Betrieb eine konstante Temperatur einzustellen, müssen laufend 25,5 kcal/Mol über einen Wärmetauscher abgeführt werden. Ohne diese Maßnahme würde die Temperatur des Reaktionsgases immer weiter bis zur vollständigen Überhitzung und zur Totaloxydation der Isobuttersäure oder zur Zerstörung des Katalysators ansteigen.

Gibt man erfindungsgemäß die Isobuttersäure in flüssiger Form bei 20°C in den Gasstrom, so werden für Verdampfung und Aufheizung des Frischgases auf die Reaktionstemperatur 37,8 kcal verbraucht, so daß nur noch 9,3 kcal über einen Wärmetauscher abgeführt werden müssen. Man kommt daher mit einem kleineren Wärmetauscher von geringerer Kühlleistung aus. Weiterhin wird ein besonderer Verdampfer für die organische Verbindung entbehrlich.

Zweckmäßig wird die flüssige organische Verbindung in den heißen Teilstrom des Reaktionsgases eingeführt, der mit dem Frischgas vermischt und in den Reaktionsraum geleitet wird. Die flüssige organische Verbindung kann mittels einer Düse zu feinen Tröpfchen versprüht werden, die rasch verdampfen. Wenn eine zusätzliche Kühlung durch einen Wärmetauscher vorgesehen ist, wird die flüssige organische Verbindung mit Vorteil nach dem Austritt aus dem Wärmetauscher in den Gasstrom eingesprüht. Man kann auch an mehreren Stellen des Gaskreislaufs die organische Verbindung einsprühen, um eine Kühlung in mehreren Teilschritten zu bewirken.

Grundsätzlich besteht auch die Möglichkeit, die zur Oxydation bzw. Dehydrierung verwendete Luft oder den Sauerstoff in flüssiger Form einzusprühen, wodurch ein weiterer Teil der Reaktionswärme verzehrt werden könnte. Allerdings ist die Wirtschaftlichkeit einer solchen Maßnahme fraglich.

Der stationär aufrechterhaltene Umsatzbereich wird in engen Grenzen gehalten. Der Umsatzunterschied vom Eingang bis zum Ausgang des Reaktionsraumes liegt vorzugsweise nicht über 25% und besonders bevorzugt zwischen 5 und 20%.

Der Umsatzgrad des aus dem Reaktionsraum austretenden Reaktionsgases kann 100% betragen, liegt aber in der Regel darunter, beispielsweise bei 50 bis 80%. Das teilumgesetzte Reaktionsgas kann in üblicher Weise in seine Bestandteile zerlegt werden, wird jedoch vorzugsweise in einem oder mehreren weiteren Reaktoren, in der Regel bei etwas anderen Betriebsbedingungen, weiter umgesetzt. Dazu kann erneut Sauerstoff zugesetzt werden. Der oder die nachfolgenden Reaktoren können ebenfalls nach dem Prinzip des Differentialreaktors aufgebaut sein. Da aber nach weitgehendem Umsatz nur noch eine gemäßigte Weiterreaktion eintritt, kann für die höheren Umsetzungsstufen ein herkömmlicher Rohrreaktor eingesetzt werden. Nach Erreichen des Endumsatzes von vorzugsweise 95 bis 100% wird in üblicher Weise durch Kondensieren, Extrahieren usw. aufgearbeitet.

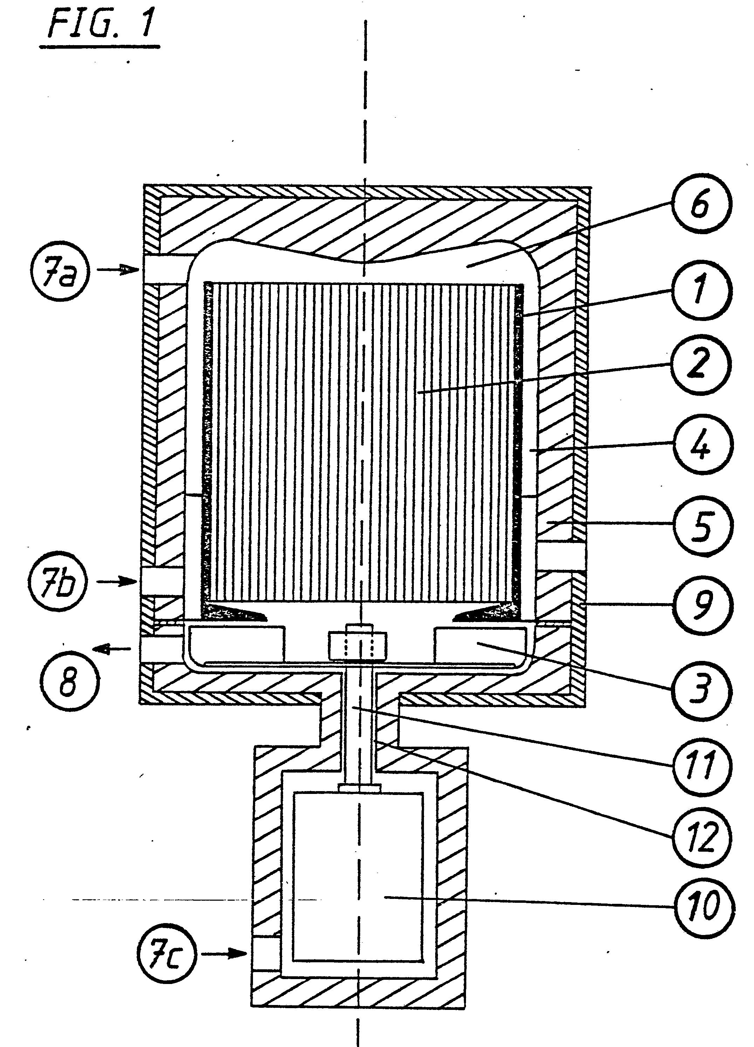

Eine zweckmäßige Gestaltung des Differentialreaktors ist in Figur 1 dargestellt. Der Katalysator 2 ist in einem Reaktionsraum 1 angeordnet und besteht aus einem zylindrischen Körper mit senkrecht durchlaufenden Wabenkanälen, deren innere Oberfläche mit der aktiven Katalysatorsubstanz beschichtet ist. Der Katalysator wird von dem mittels der Schicht 9 thermisch isolierten Reaktorgehäuse 5 so umschlossen, daß zwischen dem Reaktionsraum 1 und der Innenwandung ein ausreichender Ringraum 4 für die Rückführung des Reaktionsgases vorhanden ist. An der Eintrittsöffnung 7a oder 7b wird die umzusetzende organische Verbindung im Gaszustand eingeleitet. Sie vermischt sich im Ringraum 4 mit dem Rücklaufgas und tritt oben in den Katalysatorblock ein, der von oben nach unten durchströmt wird. Am unteren Ende des Katalysatorblockes wird das Reaktionsgas durch ein Radialgebläse 3, das über eine Welle 11 durch eine Antriebsvorrichtung 10 in Rotation versetzt wird, abgesaugt, zu einem Teil über die Austrittsöffnung 8 aus dem Reaktor entfernt und zum größeren Teil als Rücklaufgas in den Ringraum 4 zurückgeleitet. Über die Wellenbuchse 12 wird Luft, die bei 7c in das Gehäuse der Antriebsvorrichtung 10 eintritt, in den Reaktor eingeleitet. Die Erfindung ist nicht auf Reaktoren in der Bauweise gemäß Figur 1 beschränkt. Beispielsweise kann die

**0 040 666**

Rückführung des Rücklaufgases in den Reaktionsraum über eine oder mehrere Rohrleitungen außerhalb des Reaktors erfolgen. Das Gebläse kann ebenfalls außerhalb des Reaktors liegen. In jedem Falle ist die direkte Rückführung des Reaktionsgases ohne zwischengeschaltete Aufarbeitungsschritte ein wesentliches Merkmal der Erfindung.

In den nachfolgenden Beispielen wird das erfindungsgemäße Verfahren an typischen Ausführungsformen erläutert, jedoch ist das Verfahren nicht auf diese Beispiele und die verwendete Versuchsanordnung beschränkt. In allen Beispielen wurde ein Differentialreaktor in Laboratoriumsgröße mit dem in Figur 1 dargestellten Aufbau verwendet. Luft oder Sauerstoff wurde über die Leitung 7c durch das Antriebsgehäuse des Radialgebläses eingeleitet, während die gasförmige organische Verbindung durch die Leitung 7b in den Ringraum 4 eingespeist wurde. Das Reaktionsgas wurde mittels eines Radialgebläses mit einer Drehzahl von etwa 3000 Upm umgewälzt. Die Temperaturdifferenzen im Reaktionsraum lagen unter 1°C.

Für die mit einer Katalysatorkornschüttung durchgeführten Versuche wurde ein Trägermaterialgemisch aus 5 Gew.-Teilen Kieselgur und 1 Gew.-Teil hochdisperser Kieselsäure verwendet. Die Katalysatorschüttung hatte ein Volumen von 55 cm³ und eine Schichthöhe von 25 mm. Die Korngröße betrug 1,6—2,0 mm.

Bei den Versuchen an Wabenträgerkatalysatoren (Beispiel 3) wurde ein Trägerkörper aus Cordierit mit quadratischen Kanälen von 75 mm Länge und 1 qmm Querschnitt mit einer Wandstärke von etwa 0,2 mm eingesetzt.

### Beispiel 1

#### Oxydation von Methacrolein zu Methacrylsäure

Ein Gasgemisch mit einem Molverhältnis von

    1 Mol    Methacrolein
    1,25 Mol Sauerstoff
    5 Mol    Stickstoff

wurde bei 318°C über eine Katalysatorschüttung mit 70 Gew.-% einer aktiven Katalysatorsubstanz mit der Zusammensetzung $H_5Mo_{10}V_2PO_{40}$ geleitet. Die Durchsatzgeschwindigkeit betrug $5{,}95 \cdot 10^{-2}$ Mol/min · kg aktive Katalysatorsubstanz, bezogen auf Methacrolein. Der Umsatz betrug 19,5% und die Selektivität, bezogen auf Methacrylsäure 49,7%.

Mit einem Gasgemisch ohne Stickstoff wurde unter sonst gleichen Bedingungen ein Umsatz von 23,8% und eine Selektivität von 44,3% erreicht.

### Beispiel 2

#### Oxydative Dehydrierung von Isobuttersäure zu Methacrylsäure an einer Katalysatorkornschüttung

Der Katalysator enthielt 70 Gew.-% aktive Substanz der Zusammensetzung $H_5Mo_{10}V_2PO_{40}$. Die Umsetzungen wurden bei 310°C durchgeführt.

7

| Versuch | Frischgaszusammensetzung [Mol] | | | | Gasdurch-satzgeschw. $\left[\dfrac{\text{Mol IBS}}{\text{min} \cdot \text{kg KS}}\right]$ | Umsatz % bez. IBS | Selektivität für MAS in % |
|---|---|---|---|---|---|---|---|
| | IBS | $O_2$ | $N_2$ | $H_2O$ | | | |
| a | 1 | 1,5 | 10 | 2 | 0,148 | 90,4 | 74,3 |
| b | 1 | 1,5 | 6 | 0 | 0,148 | 86,1 | 75,5 |
| c | 1 | 1,5 | 6 | 0 | 0,108 | 86,7 | 76,0 |
| d | 1 | 1,5 | 3 | 0 | 0,148 | 88,9 | 71,6 |
| e | 1 | 1,5 | 0 | 0 | 0,148 | 90,3 | 63,9 |
| f | 1 | 0,5 | 0 | 0 | 0,108 | 28,2 | 51,8 |
| g | 1 | 1,0 | 0 | 0 | 0,108 | 56,3 | 59,6 |
| h | 1 | 2,0 | 0 | 0 | 0,108 | 79,6 | 60,7 |
| i | 1 | 2,5 | 0 | 0 | 0,108 | 86,4 | 58,6 |

Vergleichswerte für einen Rohrreaktor nach DE-OS 2 722 375 und DE-OS 2 633 593

| Versuch | Frischgaszusammensetzung [Mol] | | | | Gasdurch-satzgeschw. | Umsatz % bez. IBS | Selektivität für MAS in % |
|---|---|---|---|---|---|---|---|
| k*) | 1 | 1,5 | 45,5 | 2 | 5000 $h^{-1}$***) | 98 | 67,2 |
| l**) | 1 | 1,7 | 15,3 | 2 | 2000 $h^{-1}$***) | 99 | 70,2 |

IBS = Isobuttersäure.
MAS = Methacrylsäure.
KS = aktive Katalysatorsubstanz.
*) = Katalysatorzusammensetzung gemäß Beispiel A, Kat. 1 von DE-OS 2 722 375: $H_5Mo_{10}V_2PO_{40}$ auf Diatomeenerde als Träger; 310° C.
**) = Katalysatorzusammensetzung gemäß Beispiel 1, Kat. 1 von DE-OS 2 633 593: 50% $H_5Mo_{10}V_2PO_{40}$ auf Diatomeenerde als Träger; 320° C.
***) = Die Gasdurchsatzgeschwindigkeit ist in [$h^{-1}$] angegeben und wird auch als Raumge-schwindigkeit bezeichnet.

Beispiel 3

Oxydative Dehydrierung von Isobuttersäure (IBS) zu Methacrylsäure (MAS) an einem Wabenträgerkatalysator aus Cordierit mit 15,7 Gew.-% aktiver Substanz der Zusammensetzung $H_5Mo_{10}V_2PO_{40}$

Die Durchsatzgeschwindigkeit beträgt 0,148 Mol Isobuttersäure/min · kg aktive Katalysatorsub-stanz. Reaktionstemperatur 310° C.

| Ver-such | Frischgaszusammensetzung [Mol] | | | | Umsatz % bez. IBS | Selektivität für MAS in % |
|---|---|---|---|---|---|---|
| | IBS | $O_2$ | $N_2$ | $H_2O$ | | |
| a | 1 | 1,5 | 10 | 2 | 73,0 | 69 |
| b | 1 | 1,5 | 6 | 2 | 72,5 | 68 |
| c | 1 | 1,5 | 6 | 0 | 70,5 | 69 |
| d | 1 | 1,5 | 3 | 2 | 72,0 | 66 |

0 040 666

## Beispiel 4

### Oxydative Dehydrierung von Isobuttersäuremethylester (IBM) zu einem Gemisch aus Methacrylsäure (MAS) und Methacrylsäuremethylester (MMA)

Es wird eine Katalysatorkornschüttung mit 70 Gew.-% aktiver Substanz der Zusammensetzung $H_5Mo_{10}V_2PO_{40}$ verwendet. Temperatur 280 bis 316°C. Durchsatzgeschwindigkeit 4,76 · $10^{-2}$ Mol IBM pro min u. kg aktive Katalysatorsubstanz.

Die Vergleichswerte e wurden dem Beispiel B (Kat. 1) der DE-OS 2 722 375 entnommen, für das ein Katalysator gleicher Zusammensetzung auf Diatomeenerde als Träger in einem Rohrreaktor verwendet wurde. Die Durchsatzgeschwindigkeit betrug 100 h$^{-1}$.

| | Frischgaszusammensetzung [Mol] | | | Temp. | Umsatz | Selektivität in % | |
|---|---|---|---|---|---|---|---|
| | IBM | $O_2$ | $N_2$ | [°C] | % bez. IBM | für MMA | für MAS |
| Versuch | | | | | | | |
| a | 1 | 1 | 4 | 280 | 29,6 | 49,4 | 23,3 |
| b | 1 | 1,5 | 6 | 280 | 30,4 | 50,4 | 23,2 |
| c | 1 | 1,5 | 6 | 300 | 65,5 | 43,5 | 30,0 |
| d | 1 | 1,5 | 6 | 316 | 64,5 | 36,4 | 33,6 |
| Vergleich | | | | | | | |
| e | 1 | 0,84 | 29,4 | 280 | 58,1 | 45,1 | 38,8 |

## Beispiel 5

### Oxidation und Dehydrierung von Isobutryraldehyd (IBA) zu einem Gemisch aus Methacrolein (MAL) und Methacrylsäure (MAS)

Katalysatorkornschüttung mit 70 Gew.-% aktiver Substanz der Zusammensetzung $H_5Mo_{10}V_2PO_{40}$. Durchsatzgeschwindigkeit 0,07 Mol IBA/min · kg akt. Kat. Substanz.

Die Vergleichswerte e wurden dem Beispiel C (Kat. 1) der DE-OS 2 722 375 entnommen, für das ein Katalysator gleicher Zusammensetzung auf Diatomeenerde als Träger in einem Rohrreaktor eingesetzt wurde. Durchsatzgeschwindigkeit 1000 h$^{-1}$.

| | Frischgaszusammensetzung [Mol] | | | Temp. | Umsatz | Selektivität in % | |
|---|---|---|---|---|---|---|---|
| | IBA | $O_2$ | $N_2$ | [°C] | % bez. IBA | für MAL | für MAS |
| Versuch | | | | | | | |
| a | 1 | 2,7 | 10,8 | 290 | 84,5 | 7,9 | 50,7 |
| b | 1 | 1,5 | 0 | 290 | 90,8 | 10,4 | 41,7 |
| c | 1 | 1,5 | 6 | 310 | 84,7 | 12,8 | 41,4 |
| d | 1 | 2,7 | 10,8 | 310 | 85,5 | 11,9 | 40,7 |
| Vergleich | | | | | | | |
| e | 1 | 2,7 | 17,45 | 290 | 93,6 | 72,6 | 6,2 |

9

**Patentansprüche**

1. Kontinuierliches Verfahren zur Herstellung von Oxydations- oder Dehydrierungsprodukten von organischen Verbindungen durch Umsetzung eines die organische Verbindung im Gaszustand und Sauerstoff und gegebenenfalls Moderatorgas enthaltenden Frischgases mit einer unter der Umsetzungstemperatur liegenden Eintrittstemperatur durch heterogene Katalyse an einem Katalysator, der in einem Reaktionsraum angeordnet ist, zu einem Reaktionsgas, das Oxydations- bzw. Dehydrierungsprodukte der organischen Verbindung und gegebenenfalls unverbrauchten Sauerstoff enthält, wobei ein Teil des aus dem Reaktionsraum austretenden Reaktionsgases mit dem Frischgas vermischt und das entstandene Gasgemisch in den Reaktionsraum eingeleitet wird, dadurch gekennzeichnet, daß das Gemisch bzw. das Reaktionsgas mittels einer mechanisch angetriebenen Umwälzvorrichtung durch den Reaktionsraum geleitet wird und ein Frischgas mit nicht mehr als 12 Volumenteilen an Moderatorgas je Volumenteil der gasförmigen organischen Verbindung eingesetzt wird.

2. Kontinuierliches Verfahren nach Anspruch 1, dadurch gekennzeichnet, daß die Umsetzung und die Vermischung des rückgeführten Teils des Reaktionsgases mit dem Frischgas im wesentlichen adiabatisch durchgeführt wird.

3. Kontinuierliches Verfahren nach den Ansprüchen 1 und 2, dadurch gekennzeichnet, daß ein Frischgas mit nicht mehr als 8 Volumenteilen Moderatorgas je Volumenteil der gasförmigen organischen Verbindung eingesetzt wird.

4. Kontinuierliches Verfahren nach den Ansprüchen 1 bis 3, dadurch gekennzeichnet, daß ein Frischgas mit weniger als 1, vorzugsweise weniger als 0,1 Volumenteilen Wasserdampf je Volumenteil der gasförmigen organischen Verbindung eingesetzt wird.

5. Kontinuierliches Verfahren nach den Ansprüchen 1 bis 4, dadurch gekennzeichnet, daß mindestens 80 Vol.-% des Reaktionsgases mit dem Frischgas vermischt und in den Reaktionsraum zurückgeführt werden.

6. Kontinuierliches Verfahren nach den Ansprüchen 1 bis 5, dadurch gekennzeichnet, daß das Frischgas Isobutyraldehyd, Methacrolein, Isobuttersäure oder einen niederen Alkylester dieser Säure enthält und zu Methacrylsäure oder dem entsprechenden Methacrylsäurealkylester oxydiert bzw. dehydriert wird.

7. Kontinuierliches Verfahren nach den Ansprüchen 1 bis 6, dadurch gekennzeichnet, daß das Gasgemisch in dem Reaktionsraum an einem fest angeordneten Katalysator umgesetzt wird.

8. Kontinuierliches Verfahren nach Anspruch 7, dadurch gekennzeichnet, daß das Gasgemisch an einem mit im wesentlichen geradlinigen, offenen Durchströmungskanälen ausgerüsteten, sogenannten Wabenkatalysator umgesetzt wird.

9. Kontinuierliches Verfahren nach den Ansprüchen 1 bis 8, dadurch gekennzeichnet, daß der nicht in den Reaktionsraum zurückgeführte Teilstrom des Reaktionsgases, gegebenenfalls nach Zusatz von Sauerstoff, durch einen zweiten Reaktionsraum mit einem darin angeordneten Katalysator geleitet und weiter umgesetzt wird.

10. Kontinuierliches Verfahren nach den Ansprüchen 1 bis 9, dadurch gekennzeichnet, daß die organische Verbindung zum Teil oder vollständig in flüssiger Form in das Frischgas und/oder in das Reaktionsgas bzw. den mit dem Frischgas zu vermischenden Teilstrom des Reaktionsgases eingespeist und verdampft wird.

11. Verwendung einer Umsetzungsvorrichtung, bestehend aus einem Reaktionsraum mit einem darin angeordneten Katalysator, einer mechanisch angetriebenen Umwälzvorrichtung zur Beförderung eines Gasstromes durch den Reaktionsraum und einer Leitung zur Rückführung eines Teils des aus dem Reaktionsraum austretenden Reaktionsgases in den Reaktionsraum, sowie einer zum Reaktionsraum führenden Frischgaszufuhrleitung und einer aus dem Reaktionsraum herausführenden Reaktionsgasleitung, zur kontinuierlichen katalytischen Umsetzung von Isobutyraldehyd, Methacrolein, Isobuttersäure oder deren niederen Alkylestern zu Methacrylsäure bzw. deren entsprechenden niederen Alkylestern in der Gasphase nach dem Verfahren der Ansprüche 6 bis 10.

12. Verwendung einer Umsetzungsvorrichtung zur kontinuierlichen katalytischen Dehydrierung gemäß Anspruch 9, dadurch gekennzeichnet, daß der Katalysator im Reaktionsraum der Umsetzungsvorrichtung als Wabenkatalysator angeordnet ist.

**Claims**

1. Continuous process for the preparation of oxidation or dehydrogenation products of organic compounds by reaction of a carburated fuel containing the organic compound in gaseous form together with oxygen and optionally a moderator gas, at an inlet temperature below the reaction temperature, by heterogeneous catalysis using a catalyst which is provided in a reaction chamber, to form a reaction gas which contains the oxidation or dehydrogenation products of the organic compound and, optionally, any unconsumed oxygen, whilst some of the reaction gas leaving the reaction chamber is mixed with the carburated fuel and the resulting mixture of gases is introduced into the reaction chamber, characterised in that the mixture or the reaction gas is passed through the reaction chamber by means of a mechanically driven circulating apparatus and a carburated fuel containing not more than 12 parts by volume of moderator gas to each part by volume of the gaseous organic compound is used.

2. Continuous process as claimed in claim 1, characterised in that the reaction and the mixing of the recycled portion of the reaction gas with the carburated fuel is effected substantially adiabatically.

3. Continuous process as claimed in claims 1 and 2, characterised in that a carburated fuel containing not more than 8 parts by volume of moderator gas to each part by volume of the gaseous organic compound is used.

4. Continuous process as claimed in claims 1 to 3, characterised in that a carburated fuel containing less than 1, preferably less than 0.1 parts by volume of water vapour to each part by volume of gaseous organic compound is used.

5. Continuous process as claimed in claims 1 to 4, characterised in that at least 80% by volume of the reaction gas is mixed with the carburated fuel and recycled into the reaction chamber.

6. Continuous process as claimed in claims 1 to 5, characterised in that the carburated fuel contains isobutyraldehyde, methacrolein, isobutyric acid or a lower alkyl ester of this acid and is oxidised or dehydrogenated to form methacrylic acid or the corresponding alkyl methacrylate.

7. Continuous process as claimed in claims 1 to 6, characterised in that the gas mixture is reacted in the reaction chamber in the presence of a catalyst fixed therein.

8. Continuous process as claimed in claim 7, characterised in that the gas mixture is reacted in the presence of a so-called honeycomb catalyst provided with essentially straight, open flow channels.

9. Continuous process as claimed in claims 1 to 8, characterised in that the partial current of reaction gas which is not recycled into the reaction chamber is passed, optionally after the addition of oxygen, through a second reaction chamber with a catalyst provided therein and is reacted further.

10. Continuous process as claimed in claims 1 to 9, characterised in that the organic compound is fed, partly or totally in liquid form, into the carburated fuel and/or into the reaction gas or into the partial current of reaction gas which is to be mixed with the carburated fuel and is vaporised.

11. Use of a reaction apparatus consisting of a reaction chamber with a catalyst mounted therein, a mechanically driven circulating apparatus for conveying a current of gas through the reaction chamber and a line for recycling some of the reaction gas leaving the reaction chamber back into the reaction chamber, and a feed line for carburated fuel leading into the reaction chamber and a reaction gas line leading out of the reaction chamber, for the continuous catalytic reaction of isobutyraldehyde, methacrolein, isobutyric acid of a lower alkyl esters thereof to form methacrylic acid or the corresponding lower alkyl esters thereof in the gaseous phase using the process as claimed in claims 6 to 10.

12. Use of a reaction apparatus for the continuous catalytic dehydrogenation as claimed in claim 9, characterised in that the catalyst is provided in the form of a honeycomb catalyst in the reaction chamber of the reaction apparatus.

## Revendications

1. Procédé continu pour la préparation de produits d'oxydation ou de déshydrogénation de composés organiques, par la mise en réaction d'un gaz frais, contenant le composé organique à l'état gazeux, de l'oxygène et, le cas échéant, un gaz modérateur et ayant à l'entrée une température qui se situe au-dessous de la température de réaction, par catalyse hétérogène en présence d'un catalyseur qui est placé dans une zone de réaction, en vue de l'obtention d'un gaz réactionnel qui contient les produits d'oxydation ou de déshydrogénation du composé organique avec, le cas échéant, de l'oxygène non consommé, une partie du gaz réactionnel qui sort de la zone de réaction étant mélangée au gaz frais et le mélange de gaz qui en résulte étant introduit dans la zone de réaction, caractérisé en ce que le mélange ou le gaz réactionnel est envoyé à travers la zone de réaction au moyen d'un dispositif de circulation entraîné mécaniquement, et en ce que l'on utilise un gaz frais qui ne contient pas plus de 12 parties en volume de gaz modérateur par partie en volume du composé organique gazeux.

2. Procédé continu selon la revendication 1, caractérisé en ce que la réaction et le mélange de la partie recyclée du gaz réactionnel avec le gaz frais sont effectués de manière pratiquement adiabatique.

3. Procédé continu selon la revendication 1 ou 2, caractérisé en ce que l'on utilise un gaz frais qui ne contient pas plus de 8 parties en volume de gaz modérateur par partie en volume du composé organique gazeux.

4. Procédé continu selon l'une quelconque des revendications 1 à 3, caractérisé en ce que l'on utilise un gaz frais qui contient moins de 1 partie, de préférence moins de 0,1 partie en volume de vapeur d'eau par partie en volume du composé organique gazeux.

5. Procédé continu selon l'une quelconque des revendications 1 à 4, caractérisé en ce qu'au moins 80% en volume du gaz réactionnel sont mélangés au gaz frais et renvoyés dans la zone de réaction.

6. Procédé continu selon l'une quelconque des revendications 1 à 5, caractérisé en ce que le gaz frais contient de l'aldéhyde isobutyrique, de la méthacroléine, de l'acide isobutyrique ou un ester alcoylique inférieur de cet acide et est oxydé ou déshydrogéné pour l'obtention d'acide méthacrylique ou du méthacrylate d'alcoyle correspondant.

7. Procédé continu selon l'une quelconque des revendications 1 à 6, caractérisé en ce que le mélange de gaz est mis en réaction, dans la zone de réaction, en présence d'un catalyseur fixe.

8. Procédé continu selon la revendication 7, caractérisé en ce que le mélange de gaz est mis en réaction en présence d'un catalyseur dit en nid d'abeilles, muni de passages ouverts, pratiquement rectilignes.

9. Procédé continu selon l'une quelconque des revendications 1 à 8, caractérisé en ce que le courant partiel du gaz réactionnel qui n'est pas recyclé dans la zone de réaction est envoyé, le cas échéant après addition d'oxygène, à travers une seconde zone de réaction dans laquelle est disposé un catalyseur, pour y poursuivre la réaction.

10. Procédé continu selon l'une quelconque des revendications 1 à 9, caractérisé en ce que le composé organique est introduit en partie ou en totalité sous forme liquide dans le gaz frais et/ou dans le gaz réactionnel ou le courant partiel du gaz réactionnel destiné à être mélangé au gaz frais, et y est évaporé.

11. Utilisation d'un dispositif de réaction, comprenant une zone de réaction dans laquelle est disposé un catalyseur, un dispositif de mise en circulation, entraîné mécaniquement, pour faire circuler un courant gazeux à trevers la zone de réaction et une conduite pour recycler dans la zone de réaction une partie du gaz réactionnel qui sort de la zone de réaction, sinsi qu'une conduite d'arrivée de gaz frais aboutissant dans la zone de réaction et une conduite de gaz réactionnel partant de la zone de réaction, pour la transformation catalytique continue d'aldéhyde isobutyrique, de méthacroléine, d'acide isobutyrique ou des esters d'alcoyles inférieurs de celui-ci en acide méthacrylique ou en les esters alcoyliques inférieurs correspondante de celui-ci, en phase gazeuse suivant le procédé des revendications 6 à 10.

12. Utilisation d'un dispositif de réaction pour la déshydrogénation catalytique continue selon la revendication 9, caractérisée en ce que le catalyseur est disposé, dans la zone de réaction du dispositif de réaction, sous forme de catalyseur en nid d'abeilles.

*FIG. 1*